# EUROPÄISCHE PATENTSCHRIFT

(11) **EP 3 181 540 B1**
(45) Veröffentlichungstag und Bekanntmachung des Hinweises auf die Patenterteilung: **24.07.2019**
(21) Anmeldenummer: 15400056.6
(22) Anmeldetag: 18.12.2015
(51) Int. Cl.: C07C 29/76, B01D 53/22, B01D 53/14, C07C 31/04

(54) **VERFAHREN ZUR TRENNUNG VON METHANOL AUS GASGEMISCHEN**
PROCESS FOR THE SEPARATION OF METHANOL FROM GAS MIXTURES
PROCÉDÉ POUR LA SÉPARATION DE MÉTHANOL DES MÉLANGES GAZEUX

(43) Veröffentlichungstag der Anmeldung: 21.06.2017
(73) Patentinhaber: L'Air Liquide Société Anonyme pour l'Etude et l'Exploitation des Procédés Georges Claude, 75007 Paris (FR)
(72) Erfinder: Nuri, Benjamin, 61440 Oberursel (DE); Oelmann, Tobias, 61118 Bad Vilbel (DE); Ott, Jörg, 60389 Frankfurt am Main (DE); Raventos, Martin, 60439 Frankfurt am Main (DE)
(74) Vertreter: Dropsch, Holger

(56) Entgegenhaltungen:
- EP-A2- 0 478 889
- DE-A1-102009 052 640
- US-A- 3 950 369

## Beschreibung

### Gebiet der Erfindung

Die vorliegende Erfindung betrifft ein Verfahren zur Abscheidung und Wiedergewinnung von Methanol aus Gasen durch Wasserwäsche. Des weiteren betrifft die Erfindung die Verwendung einer Waschvorrichtung in dem erfindungsgemässen Verfahren.

### Stand der Technik

Verfahren zur Herstellung von Methanol durch katalytische Umsetzung von Wasserstoff und Kohlenstoffoxide enthaltendem Synthesegas sind der Fachwelt seit langer Zeit bekannt. So werden in Ullmann's Encyclopedia of Industrial Chemistry, Sixth Edition, 1998 Electronic Release, Kapitel "Methanol", Unterkapitel 5.2 "Synthesis" verschiedene Grundverfahren zur Herstellung von Methanol beschrieben.

Ein moderneres, zweistufiges Verfahren zur Herstellung von Methanol ist beispielsweise aus der EP 0 790 226 B1 bekannt. Das Methanol wird in einem Kreisprozess hergestellt, bei dem eine Mischung von frischem und teilweise reagiertem Synthesegas zunächst einem wassergekühlten Reaktor und dann einem gasgekühlten Reaktor zugeführt wird, in welchen das Synthesegas jeweils an einem kupferbasierten Katalysator zu Methanol umgesetzt wird. Das in dem Prozess hergestellte Methanol wird von dem zurückzuführenden Synthesegas abgeschieden, welches dann als Kühlmittel im Gegenstrom durch den gasgekühlten Reaktor hindurchgeführt und auf eine Temperatur von 220 bis 280°C vorgewärmt wird, bevor es in den ersten Synthesereaktor eingebracht wird. Ein Teil des zurückzuführenden Synthesegases wird als Spülstrom (sog. Purge) aus dem Verfahren entfernt, der gegenüber dem im System vorhandenen Gasinventar klein ist, um zu verhindern, dass sich Inertkomponenten, Verunreinigungen oder Nebenprodukte innerhalb des Synthesekreislaufes anreichern. Diese Maßnahme wird auch in der deutschen Offenlegungsschrift DE 2934332 A1 und der europäischen Patentanmeldung EP 1016643 A1 gelehrt.

Der aus dem Synthesekreislauf ausgeleitete Purgegasstrom ist noch mit signifikanten Anteilen an Methanol beladen. Dasselbe gilt für andere Abgasströme, die innerhalb der Methanolsynthese und der Aufarbeitung der Rohprodukte erhalten werden, wie beispielsweise Abgasen aus Methanol-Entspannungsbehältern oder Abgasen aus Lagertanks für Rohmethanol, Methanol-Wasser-Gemischen oder Reinmethanol. Unter Rohmethanol wird dabei das direkt der Methanolsynthese entstammende Primärprodukt vor der destillativen Aufarbeitung zu Reinmethanol verstanden.

Methanol-Lagertanks werden häufig als Festdachtanks ausgeführt, wobei zur Inertisierung des nicht mit Methanol ausgefüllten Innenvolumens dieses häufig mit Stickstoff gespült bzw. ausgefüllt wird. Die Stickstoffatmosphäre sättigt sich dabei mit Methanol. Bei Beladung der Tanks oder durch Tankatmung, beispielsweise durch Sonneneinstrahlung, wird mit Methanol beladener Stickstoff aus dem Tank ausgeleitet, um einen Überdruck im Tank zu vermeiden.

Die Methanol-Anteile solcher Abgase sind aufgrund des niedrigen Siedepunkts des Methanols von 65 °C bei Umgebungsdruck durchaus bedeutend, da die Abgase in der Regel bei der jeweiligen Temperatur mit Methanoldampf gesättigt sind. So beträgt unter Sättigungsbedingungen die Methanolkonzentration in einem Abgasstrom mit einer Temperatur von 42 °C rund 33 Vol.-%.

Angesichts dieser Methanolgehalte ist es plausibel, dass die Wiedergewinnung des Methanols aus den Abgasen einen wichtigen Beitrag zur Wirtschaftlichkeit des Methanolsyntheseverfahrens liefert. Andererseits ist es auch aus Umweltschutzgründen nicht vertretbar, vor dem Hintergrund der hohen Toxizität des Methanols Abgase mit derart hohen Methanolgehalten ohne Nachbehandlung an die Umwelt abzugeben. Schließlich bereiten hohe Methanolgehalte in Abgasen auch bei ihrer Weiterverarbeitung Probleme, da Methanol auskondensieren und so beispielsweise Gasbrenner beschädigen kann.

Aufgrund der hohen Wasserlöslichkeit des Methanols hat sich als Abscheidemethode die Wäsche der Abgase mit Wasser als Wachmittel bewährt. So lehrt die offengelegte europäische Patentanmeldung EP 2 168 938 A1 die Wiedergewinnung von Methanol aus Abgasen der Rohmethanoldestillation durch Wasserwäsche in einer Gegenstromkolonne. Auch die europäische Patentschrift EP 0 802 893 B1 beschreibt die Abtrennung und Wiedergewinnung von Methanol aus Abgasen der Rohmethanoldestillation durch Wasserwäsche.

Das US-Patent 5 346 593 A beschreibt eine Destillationskolonne zur Gewinnung von Reinmethanol, die an ihrem Kopf mit einer Wasserwaschstufe ausgestattet ist und zur Abscheidung von Methanolresten aus einem die Kolonne verlassenden Abgasstrom dient.

Die in einem weiteren US-Patent, US 3 950 369 A offenbarte Erfindung betrifft ein Verfahren zur Herstellung von Methanol und insbesondere eine Verbesserung der Rückgewinnung von Methanol aus einem Gasstrom, der es enthält. Gemäß der Erfindung wird Synthesegas, das Kohlendioxid enthält, über einen Methanolsynthesekatalysator geleitet, gekühlt, um einen Teil des in dem Gas enthaltenen Methanols und Wassers zu entfernen, sodann mit einer Flüssigkeit gewaschen, um im Wesentlichen den Rest dieser Stoffe zu entfernen und schließlich das Gas zu dem Methanolsynthesekatalysator zurückgeführt.

Die europäische Patentanmeldung EP 0 478 889 A2 lehrt ein Verfahren zur Verbesserung der Methanolausbeute in einer Methanolsynthese unter Einsatz eines Synthesereaktors, mindestens eines Wärmeaustauschers, eines Methanolkondensators und eines Rohmethanolabscheiders. Das offenbarte Verfahren ist dadurch gekennzeichnet, dass wenigstens ein Teil des den Synthesereaktor verlassenden Gases zu einer weiteren Wärmeabgabe einer weiteren Kondensation von Methanol und einer weiteren Methanolabscheidung zugeführt wird.

Schließlich offenbart die europäische Patentschrift EP 0 009 385 B1 die Abtrennung von Methanol aus dem aus dem Synthesekreislauf ausgeleiteten Purgegasstrom mittels Wasserwäsche und die Wiedergewinnung des abgetrennten Methanols mittels Destillation.

Bei der Verwendung von Wasser als Waschmittel bei der Abtrennung von Methanol aus Gasen ist es von Vorteil, dass sich Methanol leicht in Wasser löst. Die bei der Kondensation und der Absorption des Methanols freigesetzte Wärmemenge beeinträchtigt jedoch die Löslichkeit des Methanols in Wasser und somit die Abscheideeffizienz. Es besteht daher Bedarf an Waschvorrichtungen, die diesem Umstand Rechnung tragen.

### Beschreibung der Erfindung

Vor diesem Hintergrund besteht die Aufgabe der vorliegenden Erfindung darin, ein Verfahren bereitzustellen, bei der bzw. durch das eine effiziente Wiedergewinnung von Methanol aus Gasen, insbesondere aus Prozessabgasen der Synthese, Lagerung und Weiterverarbeitung von Methanol, sichergestellt wird.

Diese Aufgabe wird durch ein Verfahren mit den Merkmalen des Anspruchs 1 und durch die Verwendung einer Waschvorrichtung in dem beanspruchten Verfahren mit den Merkmalen des Anspruchs 8 gelöst. Weitere Ausgestaltungen der Erfindung ergeben sich aus den jeweiligen Unteransprüchen.

### Erfindungsgemäßes Verfahren:

Verfahren zur Abscheidung und Wiedergewinnung von Methanol aus Gasen durch Wasserwäsche, umfassend folgende Schritte:
(a) Bereitstellen einer Waschkolonne, mit einem Mantel, der sich - bezogen auf einen bestimmungsgemäß angeordneten Zustand der Waschkolonne - entlang einer parallel zur Vertikalen verlaufenden Längsachse erstreckt und der einen Innenraum der Waschkolonne umschließt, wobei der Innenraum in einen oberen und einen unteren Bereich unterteilt ist, die jeweils mindestens eine Stoffaustauschzone umfassen und die mittels eines Bodens, insbesondere eines Kaminbodens, voneinander getrennt werden, wobei der Boden durchlässig für eine von der unteren zur oberen Stoffaustauschzone gerichtete Gasströmung, aber undurchlässig für eine von der oberen zur unteren Stoffaustauschzone gerichtete Flüssigkeitsströmung ist und dazu geeignet ist, ein definiertes Volumen von aus der oberen Stoffaustauschzone ablaufendem Waschmittel zwischenzuspeichern, wobei die obere und die untere Stoffaustauschzone mit Stoffaustauschvorrichtungen, vorzugsweise Trennböden, Siebböden, Glockenböden, Ventilböden, strukturierten Packungen oder Füllkörperschüttungen oder Kombinationen dieser Stoffaustauschvorrichtungen ausgestattet sind,
(b) Zuführen von Wasser als Waschmittel zu einem ersten Wärmetauscher, Abkühlen des Waschmittels im ersten Wärmetauscher, Abführen eines gekühlten Waschmittelstroms aus dem ersten Wärmetauscher und Zuführen des gekühlten Waschmittelstroms in den oberen Bereich der Waschkolonne, wobei die Zugabestelle oberhalb der oberen Stoffaustauschzone angeordnet und als Flüssigkeitsverteiler ausgestaltet ist,
(c) Abführen eines mit Methanol teilbeladenen Waschmittelstroms vom Trennboden und Zuführen des mit Methanol teilbeladenen Waschmittelstroms zu einem zweiten Wärmetauscher, Abführen des mit Methanol teilbeladenen, gekühlten Waschmittelstroms aus dem zweiten Wärmetauscher und Zuführen des mit Methanol teilbeladenen, gekühlten Waschmittelstroms in den unteren Bereich der Waschkolonne, wobei die Zugabestelle unterhalb des Kaminbodens und oberhalb der unteren Stoffaustauschzone angeordnet und als Flüssigkeitsverteiler ausgestaltet ist,
(d) Ausleiten eines mit Methanol beladenen Waschmittelstroms aus dem unteren Bereich der Waschkolonne als deren Sumpfprodukt, wobei die Abzugsstelle des beladenen Waschmittelstroms unterhalb der unteren Stoffaustauschzone angeordnet ist,
(e) Zuführen eines mit Methanol beladenen Gasstroms in den unteren Bereich der Waschkolonne, wobei die Zugabestelle unterhalb der unteren Stoffaustauschzone angeordnet ist,
(f) Ausleiten eines hinsichtlich seiner Methanolbeladung reduzierten Gasstroms aus dem oberen Bereich der Waschkolonne als deren Kopfprodukt, wobei die Abzugsstelle des Gasstroms oberhalb der oberen Stoffaustauschzone angeordnet ist.

Das als Waschmittel verwendete Wasser ist zumeist demineralisiertes Wasser. Es können aber auch andere, insbesondere Wasserqualitäten höherer Reinheit, beispielsweise Reinstwasser oder destilliertes Wasser, als Waschmittel eingesetzt werden. Wasser geringerer Reinheit ist dann als Waschmittel einsetzbar, wenn die vorhandenen Begleitstoffe in nachgeschalteten Verfahrensstufen keine Probleme bereiten. So ist es möglich, bei Verwendung der erfindungsgemäßen Waschvorrichtung innerhalb einer Anlage zur Synthese von Rohmethanol und dessen Aufreinigung zu Reinmethanol auch bereits mit Methanol teilbeladene Wasserströme als Waschmittel zu verwenden, wenn die Produktspezifikationen des Reinmethanol-Produkts trotzdem eingehalten werden.

### Bevorzugte Ausgestaltungen der Erfindung

In bevorzugter Ausgestaltung umfasst die im erfindungsgemässen Verfahren verwendete Waschvorrichtung ferner eine Leitung zur Rückführung mindestens eines Teils des Sumpfprodukts der Waschkolonne zum zweiten Wärmetauscher, wobei der rückgeführte Teil des Sumpfprodukts der Waschkolonne mit dem mit Methanol teilbeladenen Waschmittelstrom zusammengeführt wird. Hierdurch wird der Durchsatz gekühlten Waschmittels im unteren Bereich der Waschkolonne und somit die Methanol-Abscheiderate erhöht.

In weiterer bevorzugter Ausgestaltung umfasst die im erfindungsgemässen Verfahren verwendete Waschvorrichtung ferner eine im oberen Bereich des Innenraums, oberhalb der oberen Stoffaustauschzone und oberhalb des oberen Flüssigkeitsverteilers angeordnete Kondensationsvorrichtung, vorzugsweise einen Rückflusskondensator. Hierdurch wird die Gesamtabscheiderate des Methanols in der Waschvorrichtung nochmals erhöht.

In bevorzugter Ausgestaltung des erfindungsgemäßen Verfahrens wird mindestens ein Teil des Sumpfprodukts der Waschkolonne zum zweiten Wärmetauscher zurückgeführt und mit dem mit Methanol teilbeladenen Waschmittelstrom zusammengeführt. Hierdurch wird der Durchsatz des Waschmittels im unteren Bereich der Waschkolonne und somit die Methanol-Abscheiderate erhöht.

In weiterer bevorzugter Ausgestaltung des erfindungsgemäßen Verfahrens erfolgt das Zusammenführen des Sumpfprodukts der Waschkolonne mit dem mit Methanol teilbeladenen Waschmittelstrom vor dem Zuführen zum zweiten Wärmetauscher. Durch das Abkühlen des vereinigten Waschmittelstroms erhöht sich abermals die Methanol-Abscheiderate.

In einem weiteren Aspekt des erfindungsgemäßen Verfahrens erfolgt eine Auskondensation von Flüssigkeit aus dem die obere Stoffaustauschzone verlassenden Gasstrom mittels einer im oberen Bereich des Innenraums, oberhalb der oberen Stoffaustauschzone und oberhalb des oberen Flüssigkeitsverteilers angeordneten Kondensationsvorrichtung, vorzugsweise einem Rückflusskondensator. Hierdurch wird die Gesamtabscheiderate des Methanols in der Waschvorrichtung nochmals erhöht.

Bevorzugt wird das erfindungsgemäße Verfahren zur Abscheidung und Wiedergewinnung von Methanol aus Abgasen von Methanollagertanks oder aus Abgasen von Methanol-Entspannungsbehältem oder aus Purgegas der Methanolsynthese oder aus Kombinationen dieser Abgase verwendet. Alle genannten Abgase enthalten signifikante Mengen Methanols in Dampfform, so dass die Abscheidung und Wiedergewinnung dieses Stoffes aus ökonomischen und ökologischen Gründen sinnvoll ist.

In bevorzugter Ausgestaltung des erfindungsgemäßen Verfahrens wird als Waschmittel demineralisiertes Wasser verwendet. Es steht in integrierten Produktionsanlagen meist als Betriebsmittel zur Verfügung und verfügt über besonders gute Absorptionseigenschaften für Methanol.

Das als Sumpfprodukt der Waschkolonne erhaltene Methanol-Wasser-Gemisch wird bevorzugt einem Methanol-Entspannungsbehälter, einem Methanol-Lagertank oder einer Destillationsvorrichtung zur Gewinnung von Reinmethanol zugeführt.

### Ausführungs- und Zahlenbeispiele

Weitere Merkmale, Vorteile und Anwendungsmöglichkeiten der Erfindung ergeben sich auch aus der nachfolgenden Beschreibung von Ausführungs- und Zahlenbeispielen und der Zeichnungen.

Es zeigen:
- Fig. 1: die schematische Darstellung des erfindungsgemäßen Verfahrens bzw. der erfindungsgemäßen Waschvorrichtung nach einer ersten Ausgestaltung,
- Fig. 2: die schematische Darstellung des erfindungsgemäßen Verfahrens bzw. der erfindungsgemäßen Waschvorrichtung nach einer zweiten Ausgestaltung,
- Fig. 3: die schematische Darstellung des erfindungsgemäßen Verfahrens bzw. der erfindungsgemäßen Waschvorrichtung nach einer dritten Ausgestaltung,
- Fig. 4: die Integration der erfindungsgemäßen Waschvorrichtung in ein Fließschema zur Aufarbeitung und Lagerung von Rohmethanol aus der Methanolsynthese,
- Fig. 5: die Integration der erfindungsgemäßen Waschvorrichtung in ein Fließschema zur Aufarbeitung und Lagerung von Rohmethanol aus der Methanolsynthese nach einer weiteren Ausgestaltung.

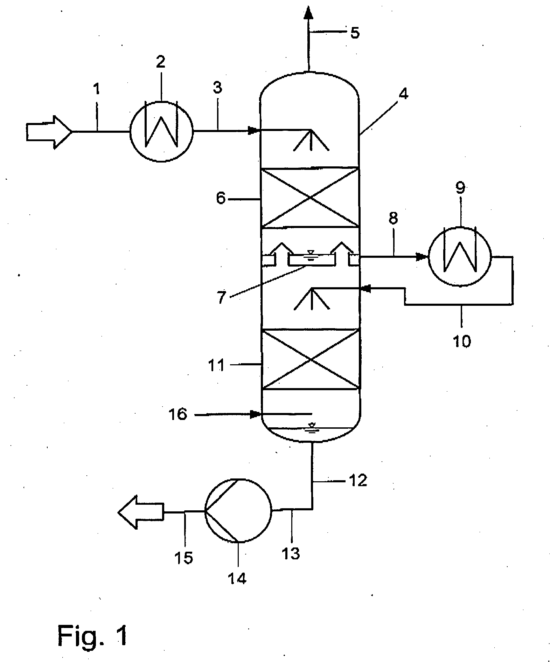

In Fig. 1 wird das erfindungsgemäße Verfahren bzw. die darin verwendete Waschvorrichtung nach einer ersten Ausgestaltung erläutert. Über die Leitung 1 wird demineralisiertes Wasser als Waschmittel zum Wärmetauscher 2 geführt und dort im indirekten Wärmetausch gegen Kühlwasser abgekühlt. Das abgekühlte Waschmittel wird über Leitung 3 zu der Waschkolonne 4 geführt und dieser durch eine in der Figur schematisch angedeutete Verteilungsvorrichtung aufgegeben. Schwerkraftvermittelt strömt das Waschmittel sodann durch die obere Stoffaustauschzone 6, bei der er sich um eine Füllkörperschüttung handelt. In der oberen Stoffaustauschzone 6 wird aufgrund der hohen spezifischen Oberfläche der Füllkörperschüttung ein intensiver Kontakt zwischen dem Waschmittel und dem zu reinigenden, mit Methanol beladenen Gasstrom hergestellt. Bei der Absorption des Methanols im Waschmittel wird die Lösungsenthalpie frei und das Waschmittel erwärmt sich entsprechend.

Nach Verlassen der oberen Stoffaustauschzone 6 gelangt das nunmehr mit Methanol teilbeladene Waschmittel zum Trennboden 7, der als Kaminboden ausgestaltet ist. Der Trennboden ist durchlässig für den aus dem unteren in den oberen Bereich des Kolonneninnenraums übertretenden Gasstrom, jedoch undurchlässig für das aus der oberen Stoffaustauschzone ablaufende Waschmittel, wodurch sich letzteres auf dem Trennboden ansammelt. Der Stand des Waschmittels auf dem Trennboden wird dabei durch die Höhe der Kamine bestimmt.

Mittels Leitung 8 wird kontinuierlich ein Teil des auf dem Trennboden 7 angesammelten, mit Methanol teilbeladenen Waschmittels aus der Waschkolonne mittels einer bildlich nicht dargestellten Fördervorrichtung abgezogen, zum Wärmetauscher 9 geführt und dort im indirekten Wärmetausch gegen Kühlwasser abgekühlt. Hierbei wird die zuvor bei der Adsorption des Methanols auf das Waschmittel übertragene Lösungsenthalpie mindestens zum Teil wieder von diesem abgeführt.

Da der über Leitung 8 aus der Waschkolonne abgezogene Waschmittelstrom dem aus der oberen Stoffaustauschzone ablaufenden Waschmittelstrom entsprechen muss, um einen konstanten Flüssigkeitsstand auf dem Trennboden sicherzustellen, muss der Wärmetauscher 9 hinsichtlich seiner Wärmeaustauschfläche und/oder der verwendeten Kühlmitteltemperatur ausgelegt werden, um eine gewünschte Temperaturabsenkung des Waschmittels zu erzielen.

Das Abziehen des auf dem Trennboden 7 angesammelten, mit Methanol teilbeladenen Waschmittels aus der Waschkolonne kann auch ohne Fördervorrichtung rein schwerkraftvermittelt erfolgen, wenn der Druckverlust in den Leitungen 8 und 10, dem Wärmetauscher 9 und der Verteilungsvorrichtung dies erlauben. In diesem Fall ist eine entsprechende Regelvorrichtung, beispielsweise ein Regelventil, vorzusehen.

Das abgekühlte Waschmittel wird über Leitung 10 zurück zu der Waschkolonne 4 geführt und dieser durch eine in der Figur schematisch angedeutete Verteilungsvorrichtung aufgegeben. Schwerkraftvermittelt strömt das Waschmittel dann durch die untere Stoffaustauschzone 11, bei der er sich wiederum um eine Füllkörperschüttung handelt. In der unteren Stoffaustauschzone 11 wird aufgrund der hohen spezifischen Oberfläche der Füllkörperschüttung ein intensiver Kontakt zwischen dem Waschmittel und dem zu reinigenden, mit Methanol beladenen Gasstrom hergestellt und somit weiteres Methanol im Waschmittel absorbiert.

Im Sinne der Erfindung können in der oberen Stoffaustauschzone 6 und in der unteren Stoffaustauschzone 11 auch andere Stoffaustauschvorrichtungen, vorzugsweise Trennböden, Siebböden, Glockenböden, Ventilböden oder strukturierte Packungen verwendet werden. Auch Kombinationen der genannten Stoffaustauschvorrichtungen sind möglich, wobei dann die Stoffaustauschzonen in entsprechende Abschnitte unterteilt werden, die jeweils mit einer der genannten Stoffaustauschvorrichtungen ausgestattet sind.

Das mit Methanol beladene Waschmittel sammelt sich im unteren Bereich der Waschkolonne als Kolonnensumpf. Aus diesem wird es über Leitungen 12 und 13, Pumpe 14 und Leitung 15 abgeführt und der Lagerung, Aufarbeitung oder einer sonstigen Verwendung zugeführt.

Über Leitung 16 wird der zu reinigende Gasstrom der Waschkolonne 4 an deren unterem Ende, zwischen Kolonnensumpf und unterer Stoffaustauschzone, aufgegeben. Es strömt dann im Gegenstrom zu dem Waschmittel zunächst durch die untere Stoffaustauschzone, in der eine Vorabscheidung des Methanols durch Absorption im Waschmittel erfolgt. Anschließend strömt der zu reinigende Gasstrom durch den Trennboden und die obere Stoffaustauschzone, in der eine Feinabscheidung des restlichen Methanols bis auf Spuren erfolgt. Der nunmehr gereinigte Gasstrom wird über Leitung 5 abgeführt und an die Umgebung abgegeben oder einer Weiterverarbeitung zugeführt.

Im vorliegenden Beispiel handelt es sich bei dem zu reinigenden Gasstrom um einen Abgasstrom aus Methanollagertanks. In diesen wird Inertgas, häufig Stickstoff, als Spülgas bzw. Überdeckungsgas verwendet, um einen Luftkontakt des gelagerten Methanols zu vermeiden. Dabei reichert sich das Inertgas mit signifikanten Mengen Methanols an.

Das erfindungsgemässe Abscheideverfahren ist ebenso für die Abscheidung von Methanol aus Abgasen von Methanol-Entspannungsbehältem oder aus Purgegas der Methanolsynthese oder aus Kombinationen der vorgenannten Abgase verwendbar.

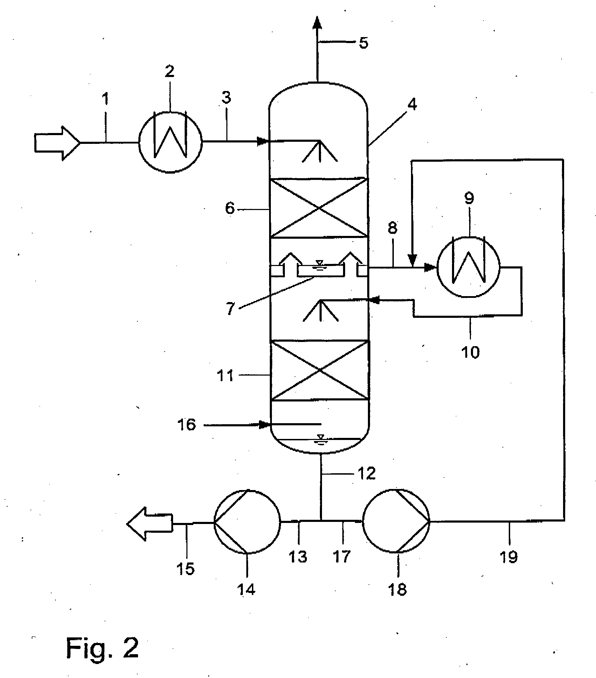

In der in Fig. 2 gezeigten, zweiten Ausgestaltung des erfindungsgemäßen Verfahrens wird in Ergänzung der in Fig. 1 dargestellten Ausgestaltungsform ein Teil des aus dem Kolonnensumpf abgeführten, mit Methanol beladenen Waschmittels über Leitung 17, Pumpe 18 und Leitung 19 zum Wärmetauscher 9 zurückgeführt und diesem gemeinsam mit dem über Leitung 8 aus der Waschkolonne 4 abgeführten Waschmittel aufgegeben. Auf diese Weise wird der Durchsatz des Waschmittels im unteren Bereich der Waschkolonne und somit die Methanol-Abscheiderate erhöht. Durch das Abkühlen des vereinigten Waschmittelstroms aus den Leitungen 8 und 19 erhöht sich die Methanol-Abscheiderate noch weiter.

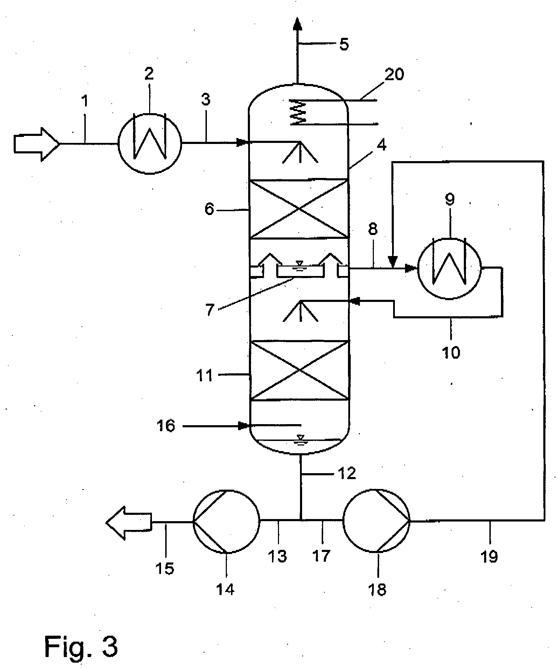

In der in Fig. 3 gezeigten, dritten Ausgestaltung des erfindungsgemäßen Verfahrens ist die Waschkolonne 4 in Ergänzung der in Fig. 2 dargestellten Ausgestaltungsform mit einer zusätzlichen im oberen Bereich des innenraums, oberhalb der oberen Stoffaustauschzone und oberhalb des oberen Flüssigkeitsverteilers angeordneten Kondensationsvorrichtung, vorzugsweise einem Rückflusskondensator, ausgestattet. Hierdurch wird die Gesamtabscheiderate des Methanols in der Waschvorrichtung nochmals erhöht und der über Leitung 5 abgeführte Gasstrom von letzten Methanolresten befreit. Die Verwendung der Kondensationsvorrichtung ist dabei auch in der in Fig. 1 gezeigten Ausgestaltung der Erfindung möglich, also ohne Rückführung von beladenem Waschmittel über Leitung 19. Die Gesamtabscheiderate des Methanols ist dabei etwas geringer als in der in Fig. 3 gezeigten Ausgestaltung.

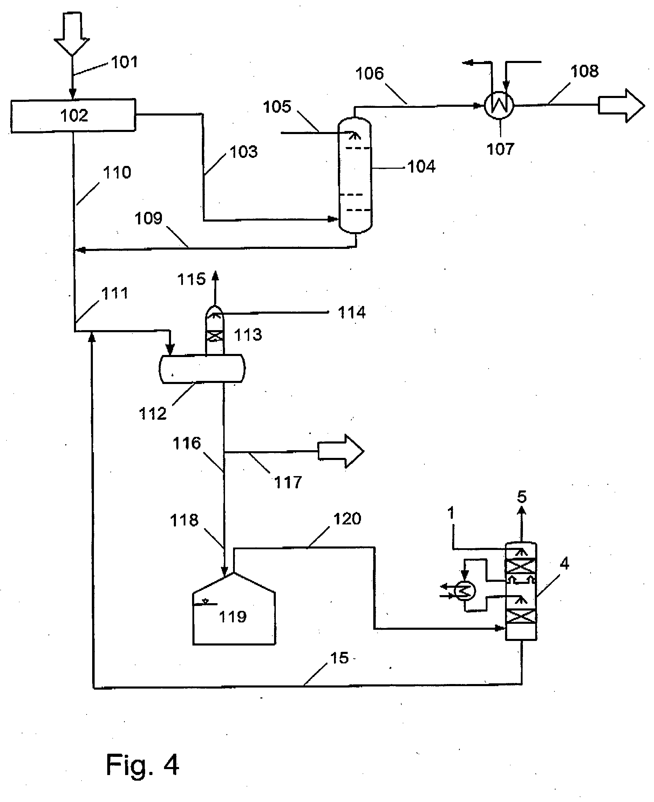

In Fig. 4 ist die Integration der im erfindungsgemässen Verfahren verwendeten Waschvorrichtung in ein Fließschema zur Aufarbeitung und Lagerung von Rohmethanol aus der Methanolsynthese dargestellt.

Über Leitung 101 wird aus Wasserstoff und Kohlenoxiden bestehendes Synthesegas in den hier nur schematisch dargestellten und nicht weiter im Detail erläuterten Methanolsynthesereaktor 102 eingeleitet, in dem das Synthesegas unter Bedingungen der Methanolsynthese teilweise zu Methanol umgesetzt wird. Das dabei erzeugte Rohmethanol wird über Leitung 10 aus dem Methanolsynthesereaktor ausgeleitet.

Der größte Teil des bei der Methanolsynthese nicht umgesetzten Synthesegases wird über einen bildlich nicht dargestellten Synthesegaskreislauf zum Eingang des Methanolsynthesereaktors zurückgeführt. Der verbliebene Anteil des nicht umgesetzten Synthesegases wird als Purgegas oder Spülgas über Leitung 103 aus dem Methanolsynthesereaktor ausgeleitet und zu der Purgegaswaschvorrichtung 104 geführt. Es handelt sich dabei um eine an sich bekannte Waschkolonne, die mit Böden, Füllkörperschüttungen oder strukturierten Packungen ausgerüstet sein kann, um den Stoffaustausch zwischen Gas und Flüssigkeit zu intensivieren. Der Purgegaswaschvorrichtung wird der Purgegasstrom an deren Unterseite aufgegeben und im Gegenstrom in Kontakt mit einem über Leitung 105 als Waschmittel herangeführten Wasserstrom gebracht, wodurch sein Gehalt an Methanoldampf reduziert wird. Im vorliegenden und in den nachfolgend beschriebenen Ausführungsbeispielen wird - sofern nicht anders vermerkt - demineralisiertes Wasser als Waschmittel verwendet.

Das an Methanol abgereicherte Purgegas verlässt über Leitung 106 die Purgegaswaschvorrichtung. Es kann dann optional in Wärmetauscher 107 im indirekten Wärmetausch gegen Niederdruckdampf als Heizmedium erhitzt und über Leitung 108 aus dem Verfahren ausgeleitet und zu einer bildlich nicht dargestellten Wasserstoffrückgewinnungsanlage geführt werden.

Das in der Purgegaswaschvorrichtung mit Methanol beladene Wasser wird über Leitung 109 aus dieser ausgeleitet und gemeinsam mit dem über Leitung 110 herangeführten Rohmethanol über Leitung 111 zu dem Entspannungsbehälter 112 geführt. In diesem wird das Rohmethanol-Wasser-Gemisch von 7,0 MPa.g auf einen Druck von 0,5 MPa,g entspannt (,g bezeichnet die entsprechende Druckeinheit bei Überdruck). Die bei der Entspannung freiwerdenden Gase bzw. Dämpfe werden in der baulich mit dem Entspannungsbehälter verbundenen und mit diesem in Fluidverbindung stehenden Entspannungsgaswaschvorrichtung 113 in Kontakt mit einem über Leitung 114 als Waschmittel herangeführten Wasserstrom gebracht, wodurch sein Gehalt an Methanoldampf reduziert wird. Auch bei der Entspannungsgaswaschvorrichtung handelt es sich um eine an sich bekannte Waschkolonne, die mit Böden, Füllkörperschüttungen oder strukturierten Packungen ausgerüstet sein kann, um den Stoffaustausch zwischen Gas und Flüssigkeit zu intensivieren. Der an Methanol abgereicherte Entspannungsgasstrom verlässt über Leitung 115 die Entspannungsgaswaschvorrichtung.

Das entspannte Rohmethanol-Wasser-Gemisch wird über Leitung 116 aus dem Entspannungsbehälter 112 ausgeleitet. Über Leitung 117 kann es vollständig oder teilweise zu einer bildlich nicht dargestellten Destillationsvorrichtung geführt werden, in der das Rohmethanol durch destillative Wasserabtrennung zu Reinmethanol weiterverarbeitet wird.

Der nicht zu der Destillationsvorrichtung geführte Anteil des Rohmethanol-Wasser-Gemischs wird über Leitung 118 zum Tank 119 geführt und in diesen eingeleitet. Aus dem Tank kann zu einem späteren Zeitpunkt Rohmethanol entnommen und der destillativen Aufarbeitung zugeführt werden. Bei dem Tank 119 handelt es sich um einen Festdachtank, in dem das freie Innenvolumen des Tanks durch Stickstoff, der über eine nicht bildlich gezeigte Leitung herangeführt wird, ausgefüllt und somit inertisiert wird. Die Stickstoffatmosphäre sättigt sich dabei mit Methanol. Bei Beladung der Tanks oder durch Tankatmung, beispielsweise durch Sonneneinstrahlung, wird mit Methanol beladener Stickstoff aus dem Tank über Leitung 120 ausgeleitet, um einen Überdruck im Tank zu vermeiden. Dieser mit Methanoldampf beladene Inertisierungsgasstrom wird zu der Waschvorrichtung 4 geführt, dieser an ihrer Unterseite aufgegeben und im Gegenstrom in Kontakt mit einem über Leitung 1 als Waschmittel herangeführten Wasserstrom gebracht, wodurch sein Gehalt an Methanoldampf reduziert wird.

Über Leitung 15 wird das mit Methanol beladene Waschmittel aus der Inertisierungsgaswaschvorrichtung ausgeleitet und über Leitung 111 zu dem Entspannungsbehälter 12 geführt. Falls in den Leitungen 15 und 111 deutlich unterschiedliche Drücke herrschen, kann das mit Methanol beladene Waschmittel auch getrennt von der Leitung 111 zu dem Entspannungsbehälter geführt werden.

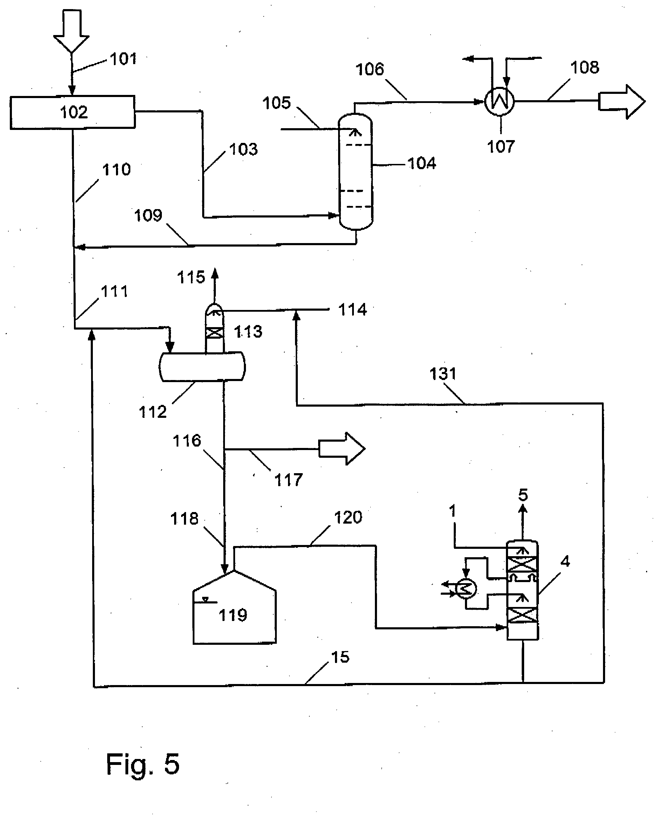

In Fig. 5 ist die Integration der erfindungsgemäßen Waschvorrichtung in ein Fließschema zur Aufarbeitung und Lagerung von Rohmethanol aus der Methanolsynthese nach einer weiteren Ausgestaltung dargestellt.

Das erfindungsgemäße Verfahren entspricht bis zu der Anlagenkomponente 120 der in Fig. 4 dargestellten Ausgestaltung. Es tritt aber nun die Leitung 131 hinzu, mit der mindestens ein Teil des über Leitung 15 aus der Waschvorrichtung abgeführten Waschmittels über Leitung 114 zu der Entspannungsgaswaschvorrichtung 113 geführt wird. Durch die in Fig. 5 gezeigte Ausgestaltung der Erfindung kann ein besonders großer Anteil des in der Entspannungsgaswaschvorrichtung als Waschmittel verwendeten, demineralisierten Wassers durch mit Methanol beladene Wasserströme aus der Waschvorrichtung 4 ersetzt werden.

In Fig. 4 und 5 ist die Waschvorrichtung jeweils nur schematisch angedeutet. Es wird daher darauf hingewiesen, dass bei ihrer Integration in ein Fließschema zur Aufarbeitung und Lagerung von Rohmethanol aus der Methanolsynthese verschiedene Ausgestaltungen der Waschvorrichtung verwendet werden können, insbesondere auch die in Fig. 1, Fig. 2 und Fig. 3 dargestellten Ausgestaltungen.

### Zahlenbeispiele

Im nachfolgenden Zahlenbeispiel wurde die Abscheidung von Methanol aus dem Inertisierungsabgas einer Methanol-Tankfarm zugrunde gelegt. Das auf Stickstoff basierende Inertisierungsgas war bei 42 °C mit Methanol gesättigt. Der Volumenstrom des Abgases betrug jeweils 100000 SCFH, entsprechend 2831,68 m³/h. Die Zusammensetzung des Inertisierungsabgases war wie folgt:
Stickstoff: 66,75 , Wasser: 0,008 Vol.-%, Methanol: 33,17 Vol.-%, Methyl-Ethyl-Keton (MEK): 2,4 Vol.-ppm, Ethanol: 1,2 Vol.-ppm, 2-Propanol: 0,3 Vol.-ppm
In allen nachfolgend dargestellten Fällen wurde als frisches Waschmittel demineralisiertes Wasser mit einem Massenstrom von 2900 kg/h und einer Temperatur von 40 °C eingesetzt. Die verwendete Waschkolonne wies jeweils dieselbe Zahl theoretischer Böden auf und war mit einem Kaminboden als Trennboden ausgestattet. Als Kühlmedium wurde jeweils demineralisiertes Wasser mit 25 Gew.-% Monoethylenglykol als Frostschutzmittel eingesetzt, wobei die Vorlauftemperatur jeweils 5 °C und die Rücklauftemperatur jeweils 15 °C betrug.

In der nachfolgenden Tabelle sind Zahlenbeispiele für die in Fig. 1, Fig. 2 und Fig. 3 gezeigten Ausgestaltungen der Erfindung zusammengestellt.

Wie der Tabelle entnommen werden kann, beträgt der Methanol-Gesamtabscheidegrad in allen drei Ausgestaltungen der Erfindung über 99 %. Den höchsten Methanol-Gesamtabscheidegrad weist die Ausgestaltung nach Fig. 3 mit 99,98 % auf. Dabei ist der Gesamt-Kühlmittelverbrauch gegenüber der Ausgestaltung nach Fig. 2 sogar leicht verringert.

| **Ausgestaltung** | | **Figur 1** | **Figur 2** | **Figur 3** |
|---|---|---|---|---|
| | | ohne Rückführung Leitung 19 | mit Rückführung Leitung 19 | mit Rückführung Leitung 19 und Kondensator 20 |
| **Inertisierungsabgas** | | | | |
| | | | | |
| Methanol-Massenstrom | kg/h | 1270.6 | 1270,6 | 1270,6 |
| Zusammensetzung: | | | | |
| Stickstoff | Vol.% | | 66.75 | |
| Wasser | Vol.% | | 0,008 | |
| Methanol | Vol.-% | | 33,17 | |
| MEK | Vol.-ppm | | 2,4 | |
| Ethanol | Vol.-ppm | | 1,2 | |
| 2-Propanol | Vol.-ppm | | 0,3 | |
| | | | | |
| Volumenstrom | SCFH | | 100,000 | |
| Temperatur | °C | | 42,0 | |

| **Gereinigtes Abgas** | | | | |
|---|---|---|---|---|
| Temperatur | °C | 52,2 | 20,5 | 15,0 |
| Methanol-Massenstrom | kg/h | 10,8121 | 0,4087 | 0,2434 |
| Zusammensetzung: | | | | |
| Stickstoff | Vol.-% | 86,27 | 97,64 | 98,33 |
| Wasser | Vol.-% | 13,37 | 2,35 | 1,66 |
| Methanol | Vol.-ppm | **3649** | **156,1** | **93,6** |
| MEK | Vol.-ppm | 3,1 | 3,5 | 3,5 |
| Ethanol | Vol.-ppm | 1,5 | 1,5 | 1,5 |
| 2-Propanol | Vol.-ppm | 0.3 | 0,4 | 0.4 |

| **Methanol-Gesamtabscheidegrad** | % | **99,15%** | **99,97%** | **99.98%** |
|---|---|---|---|---|
| Massenstrom Leitung 19 | kg/h | 0 | 8500 | 3600 |
| **Kühlmittelverbrauch** (Bezugsz.) | | | | |
| 2 | kg/h | 8236 | 8236 | 8236 |
| 9 | kg/h | 13175 | 34866 | 28131 |
| 20 | kg/h | 0 | 0 | 6653 |
| Gesamt | kg/h | **21411** | **43102** | **43020** |

| **Wärmetauscher** (Bezugsz.) | U*A | | | |
|---|---|---|---|---|
| 2 | W/°C | 6580 | 6580 | 6580 |
| 9 | W/°C | 8260 | 26400 | 17800 |
| 20 | W/°C | 0 | 0 | 5540 |
| Gesamt | W/°C | **14840** | **32980** | **29920** |

### Gewerbliche Anwendbarkeit

Mit der Erfindung wird ein Verfahren zur effizienten Abscheidung und Wiedergewinnung von Methanol aus mit Methanol beladenen Abgasen vorgeschlagen, wobei die Erfindung auch in einem integrierten Fließschema zu Herstellung und Aufarbeitung von Methanol eingesetzt werden kann. Die dabei aus den Abgasen abgeschiedenen Methanolanteile werden innerhalb der bereits bestehenden, destillativen Aufarbeitung des Rohmethanols zu Reinmethanol zurückgewonnen, so dass keine gesonderten Vorrichtungen zur Rückgewinnung des Methanols aus den beladenen Wäscherabwässern benötigt werden. Der Wertstoff Methanol wird zurückgewonnen und die Belastung der Umwelt reduziert. Durch besondere Ausgestaltungen der Erfindung kann der Methanol-Gesamtabscheidegrad gemäß den lokal geltenden Emissionsgrenzwerten angepasst werden.

### Bezugszeichenliste

- 1: Leitung
- 2: Wärmetauscher
- 3: Leitung
- 4: Waschkolonne
- 5: Leitung
- 6: obere Stoffaustauschzone
- 7: Trennboden
- 8: Leitung
- 9: Wärmetauscher
- 10: Leitung
- 11: untere Stoffaustauschzone
- 12: Leitung
- 13: Leitung
- 14: Pumpe
- 15: Leitung
- 16: Leitung
- 17: Leitung
- 18: Pumpe
- 19: Leitung
- 20: Kondensationsvorrichtung
- 101: Leitung
- 102: Methanolsynthesereaktor
- 103: Leitung
- 104: Purgegaswaschvorrichtung
- 105: Leitung
- 106: Leitung
- 107: Wärmetauscher
- 108: Leitung
- 109: Leitung
- 110: Leitung
- 111: Leitung
- 112: Entspannungsbehälter
- 113: Entspannungsgaswaschvorrichtung
- 114: Leitung
- 115: Leitung
- 116: Leitung
- 117: Leitung
- 118: Leitung
- 119: Tank
- 120: Leitung
- 131: Leitung

## Patentansprüche

1. Verfahren zur Abscheidung und Wiedergewinnung von Methanol aus Gasen durch Wasserwäsche, umfassend folgende Schritte:
(a) Bereitstellen einer Waschkolonne, mit einem Mantel, der sich - bezogen auf einen bestimmungsgemäß angeordneten Zustand der Waschkolonne - entlang einer parallel zur Vertikalen verlaufenden Längsachse erstreckt und der einen Innenraum der Waschkolonne umschließt, wobei der Innenraum in einen oberen und einen unteren Bereich unterteilt ist, die jeweils mindestens eine Stoffaustauschzone umfassen und die mittels eines Bodens, insbesondere eines Kaminbodens, voneinander getrennt werden, wobei der Boden durchlässig für eine von der unteren zur oberen Stoffaustauschzone gerichtete Gasströmung, aber undurchlässig für eine von der oberen zur unteren Stoffaustauschzone gerichtete Flüssigkeitsströmung ist und dazu geeignet ist, ein definiertes Volumen von aus der oberen Stoffaustauschzone ablaufendem Waschmittel zwischenzuspeichern, wobei die obere und die untere Stoffaustauschzone mit Stoffaustauschvorrichtungen, vorzugsweise Trennböden, Siebböden, Glockenböden, Ventilböden, strukturierten Packungen oder Füllkörperschüttungen oder Kombinationen dieser Stoffaustauschvorrichtungen ausgestattet sind,
(b) Zuführen von Wasser als Waschmittel zu einem ersten Wärmetauscher, Abkühlen des Waschmittels im ersten Wärmetauscher, Abführen eines gekühlten Waschmittelstroms aus dem ersten Wärmetauscher und Zuführen des gekühlten Waschmittelstroms in den oberen Bereich der Waschkolonne, wobei die Zugabestelle oberhalb der oberen Stoffaustauschzone angeordnet und als Flüssigkeitsverteiler ausgestaltet ist,
(c) Abführen eines mit Methanol teilbeladenen Waschmittelstroms vom Trennboden und Zuführen des mit Methanol teilbeladenen Waschmittelstroms zu einem zweiten Wärmetauscher, Abführen des mit Methanol teilbeladenen, gekühlten Waschmittelstroms aus dem zweiten Wärmetauscher und Zuführen des mit Methanol teilbeladenen, gekühlten Waschmittelstroms in den unteren Bereich der Waschkolonne, wobei die Zugabestelle unterhalb des Kaminbodens und oberhalb der unteren Stoffaustauschzone angeordnet und als Flüssigkeitsverteiler ausgestaltet ist,
(d) Ausleiten eines mit Methanol beladenen Waschmittelstroms aus dem unteren Bereich der Waschkolonne als deren Sumpfprodukt, wobei die Abzugsstelle des beladenen Waschmittelstroms unterhalb der unteren Stoffaustauschzone angeordnet ist,
(e) Zuführen eines mit Methanol beladenen Gasstroms in den unteren Bereich der Waschkolonne, wobei die Zugabestelle unterhalb der unteren Stoffaustauschzone angeordnet ist,
(f) Ausleiten eines hinsichtlich seiner Methanolbeladung reduzierten Gasstroms aus dem oberen Bereich der Waschkolonne als deren Kopfprodukt, wobei die Abzugsstelle des Gasstroms oberhalb der oberen Stoffaustauschzone angeordnet ist.

2. Verfahren nach Anspruch 1, **dadurch gekennzeichnet, dass** mindestens ein Teil des Sumpfprodukts der Waschkolonne zum zweiten Wärmetauscher zurückgeführt und mit dem mit Methanol teilbeladenen Waschmittelstrom zusammengeführt wird.

3. Verfahren nach Anspruch 2, **dadurch gekennzeichnet, dass** das Zusammenführen des Sumpfprodukts der Waschkolonne mit dem mit Methanol teilbeladenen Waschmittelstrom vor dem Zuführen zum zweiten Wärmetauscher erfolgt.

4. Verfahren nach einem der vorigen Ansprüche, **dadurch gekennzeichnet, dass** eine Auskondensation von Flüssigkeit aus dem die obere Stoffaustauschzone verlassenden Gasstrom mittels einer im oberen Bereich des Innenraums, oberhalb der oberen Stoffaustauschzone und oberhalb des oberen Flüssigkeitsverteilers angeordneten Kondensationsvorrichtung, vorzugsweise einem Rückflusskondensator, erfolgt.

5. Verfahren nach einem der vorigen Ansprüche, **dadurch gekennzeichnet, dass** es zur Abscheidung und Wiedergewinnung von Methanol aus Abgasen von Methanollagertanks oder aus Abgasen von Methanol-Entspannungsbehältern oder aus Purgegas der Methanolsynthese oder aus Kombinationen dieser Abgase verwendet wird.

6. Verfahren nach einem der vorigen Ansprüche, **dadurch gekennzeichnet, dass** als Waschmittel demineralisiertes Wasser verwendet wird.

7. Verfahren nach einem der vorigen Ansprüche, **dadurch gekennzeichnet, dass** das Sumpfprodukt der Waschkolonne einem Methanol-Entspannungsbehälter, einem Methanol-Lagertank oder einer Destillationsvorrichtung zur Gewinnung von Reinmethanol zugeführt wird.

8. Verwendung einer Waschvorrichtung, umfassend folgende Bestandteile und Baugruppen:
(a) eine Waschkolonne, mit einem Mantel, der sich - bezogen auf einen bestimmungsgemäß angeordneten Zustand der Waschkolonne - entlang einer parallel zur Vertikalen verlaufenden Längsachse erstreckt und der einen Innenraum der Waschkolonne umschließt, wobei der Innenraum in einen oberen und einen unteren Bereich unterteilt ist, die jeweils mindestens eine Stoffaustauschzone umfassen und die mittels eines Trennbodens, insbesondere eines Kaminbodens, voneinander getrennt werden, wobei der Boden durchlässig für eine von der unteren zur oberen Stoffaustauschzone gerichtete Gasströmung, aber undurchlässig für eine von der oberen zur unteren Stoffaustauschzone gerichtete Flüssigkeitsströmung ist und dazu geeignet ist, ein definiertes Volumen von aus der oberen Stoffaustauschzone ablaufendem Waschmittel zwischenzuspeichern, wobei die obere und die untere Stoffaustauschzone mit Stoffaustauschvorrichtungen, vorzugsweise Trennböden, Siebböden, Glockenböden, Ventilböden, strukturierten Packungen oder Füllkörperschüttungen oder Kombinationen dieser Stoffaustauschvorrichtungen ausgestattet sind,
(b) einen ersten Wärmetauscher, eine Leitung zum Zuführen von Wasser als Waschmittel zum ersten Wärmetauscher, eine Leitung zum Abführen eines gekühlten Waschmittelstroms aus dem ersten Wärmetauscher und zum Zuführen des gekühlten Waschmittelstroms in den oberen Bereich der Waschkolonne, wobei die Zugabestelle oberhalb der oberen Stoffaustauschzone angeordnet und als Flüssigkeitsverteiler ausgestaltet ist,
(c) einen zweiten Wärmetauscher, eine Leitung zum Abführen eines mit Methanol teilbeladenen Waschmittelstroms vom Trennboden und zum Zuführen des mit Methanol teilbeladenen Waschmittelstroms zum zweiten Wärmetauscher, eine Leitung zum Abführen des mit Methanol teilbeladenen, gekühlten Waschmittelstroms aus dem zweiten Wärmetauscher und zum Zuführen des mit Methanol teilbeladenen, gekühlten Waschmittelstroms in den unteren Bereich der Waschkolonne, wobei die Zugabestelle unterhalb des Kaminbodens und oberhalb der unteren Stoffaustauschzone angeordnet und als Flüssigkeitsverteiler ausgestaltet ist,
(d) eine Leitung zum Ausleiten eines mit Methanol beladenen Waschmittelstroms aus dem unteren Bereich der Waschkolonne als deren Sumpfprodukt, wobei die Abzugsstelle des beladenen Waschmittelstroms unterhalb der unteren Stoffaustauschzone angeordnet ist,
(e) eine Leitung zum Zuführen eines mit Methanol beladenen Gasstroms in den unteren Bereich der Waschkolonne, wobei die Zugabestelle unterhalb der unteren Stoffaustauschzone angeordnet ist,
(f) eine Leitung zum Ausleiten eines hinsichtlich seiner Methanolbeladung reduzierten Gasstroms aus dem oberen Bereich der Waschkolonne als deren Kopfprodukt, wobei die Abzugsstelle des Gasstroms oberhalb der oberen Stoffaustauschzone angeordnet ist,
in einem Verfahren gemäß Anspruch 1 bis 7 zur Abscheidung von Methanol aus Gasen durch Wasserwäsche.

9. Verwendung nach Anspruch 8, wobei die Waschvorrichtung ferner umfasst:
- eine Leitung zur Rückführung mindestens eines Teils des Sumpfprodukts der Waschkolonne zum zweiten Wärmetauscher, wobei der rückgeführte Teil des Sumpfprodukts der Waschkolonne mit dem mit Methanol teilbeladenen Waschmittelstrom zusammengeführt wird.

10. Verwendung nach Anspruch 8 oder 9, wobei die Waschvorrichtung ferner umfasst:
- eine im oberen Bereich des Innenraums, oberhalb der oberen Stoffaustauschzone und oberhalb des oberen Flüssigkeitsverteilers angeordnete Kondensationsvorrichtung, vorzugsweise einen Rückflusskondensator.

## Claims

1. A process for the separation and recovery of methanol from gases by water washing, comprising the following steps:
(a) providing a washing column, comprising a jacket which - based on a properly arranged condition of the washing column - extends along a longitudinal axis extending parallel to the vertical and which encloses an interior space of the washing column, wherein the interior space is divided into an upper and a lower region, which each comprise at least one mass transfer zone and which are separated from each other by means of a tray, in particular a chimney tray, wherein the tray is permeable for a gas flow directed from the lower to the upper mass transfer zone, but is impermeable for a liquid flow directed from the upper to the lower mass transfer zone and is suitable to temporarily store a defined volume of washing agent flowing out of the upper mass transfer zone, wherein the upper and the lower mass transfer zone are equipped with mass transfer devices, preferably separation trays, sieve trays, bubble trays, valve trays, structured packings or packed beds or combinations of these mass transfer devices,
(b) supplying water as washing agent to a first heat exchanger, cooling the washing agent in the first heat exchanger, discharging a cooled stream of washing agent from the first heat exchanger and supplying the cooled stream of washing agent into the upper region of the washing column, wherein the addition point is arranged above the upper mass transfer zone and is designed as liquid distributor,
(c) discharging a stream of washing agent partly loaded with methanol from the separation tray and supplying the stream of washing agent partly loaded with methanol to a second heat exchanger, discharging the cooled stream of washing agent partly loaded with methanol from the second heat exchanger and supplying the cooled stream of washing agent partly loaded with methanol into the lower region of the washing column, wherein the addition point is arranged below the chimney tray and above the lower mass transfer zone and is designed as liquid distributor,
(d) discharging a stream of washing agent loaded with methanol from the lower region of the washing column as its bottom product, wherein the withdrawal point of the loaded stream of washing agent is arranged below the lower mass transfer zone,
(e) supplying a gas stream loaded with methanol into the lower region of the washing column, wherein the addition point is arranged below the lower mass transfer zone,
(f) discharging a gas stream reduced in its methanol loading from the upper region of the washing column as its top product, wherein the withdrawal point of the gas stream is arranged above the upper mass transfer zone.

2. The process according to claim 4, **characterized in that** at least a part of the bottom product of the washing column is recirculated to the second heat exchanger and combined with the stream of washing agent partly loaded with methanol.

3. The process according to claim 5, **characterized in that** combining the bottom product of the washing column with the stream of washing agent partly loaded with methanol is effected before supplying the same to the second heat exchanger.

4. The process according to any of the preceding claims, **characterized in that** a condensation of liquid out of the gas stream leaving the upper mass transfer zone is effected by means of a condensation apparatus arranged in the upper region of the interior space, above the upper mass transfer zone and above the upper liquid distributor, preferably a reflux condenser.

5. The process according to any of the preceding claims, **characterized in that** it is used for the separation and recovery of methanol from waste gases of methanol storage tanks or from waste gases of methanol expansion tanks or from purge gas of the methanol synthesis or from combinations of these waste gases.

6. The process according to any of the previous claims, **characterized in that** demineralized water is used as washing agent.

7. The process according to any of the preceding claims, **characterized in that** the bottom product of the washing column is supplied to a methanol expansion tank, a methanol storage tank or a distillation apparatus for obtaining pure methanol.

8. Use of a washing apparatus comprising the following components and assemblies:
(a) a washing column, comprising a jacket which - based on a properly arranged condition of the washing column - extends along a longitudinal axis extending parallel to the vertical and which encloses an interior space of the washing column, wherein the interior space is divided into an upper and a lower region, which each comprise at least one mass transfer zone and which are separated from each other by means of a separation tray, in particular a chimney tray, wherein the tray is permeable for a gas flow directed from the lower to the upper mass transfer zone, but is impermeable for a liquid flow directed from the upper to the lower mass transfer zone and is suitable to temporarily store a defined volume of washing agent flowing out of the upper mass transfer zone, wherein the upper and the lower mass transfer zone are equipped with mass transfer devices, preferably separation trays, sieve trays, bubble trays, valve trays, structured packings or packed beds or combinations of these mass transfer devices,
(b) a first heat exchanger, a conduit for supplying water as washing agent to the first heat exchanger, a conduit for discharging a cooled stream of washing agent from the first heat exchanger and for supplying the cooled stream of washing agent into the upper region of the washing column, wherein the addition point is arranged above the upper mass transfer zone and is designed as liquid distributor,
(c) a second heat exchanger, a conduit for discharging a stream of washing agent partly loaded with methanol from the separation tray and for supplying the stream of washing agent partly loaded with methanol to the second heat exchanger, a conduit for discharging the cooled stream of washing agent partly loaded with methanol from the second heat exchanger and for supplying the cooled stream of washing agent partly loaded with methanol into the lower region of the washing column, wherein the addition point is arranged below the chimney tray and above the lower mass transfer zone and is designed as liquid distributor,
(d) a conduit for discharging a stream of washing agent loaded with methanol from the lower region of the washing column as its bottom product, wherein the withdrawal point of the loaded stream of washing agent is arranged below the lower mass transfer zone,
(e) a conduit for supplying a gas stream loaded with methanol into the lower region of the washing column, wherein the addition point is arranged below the lower mass transfer zone,
(f) a conduit for discharging a gas stream reduced in its methanol loading from the upper region of the washing column as its top product, wherein the withdrawal point of the gas stream is arranged above the upper mass transfer zone,
in a process according to claim 1 to 7 for the separation of methanol from gases by water washing.

9. Use according to claim 8, wherein the washing apparatus furthermore comprises a conduit for the recirculation of at least a part of the bottom product of the washing column to the second heat exchanger, wherein the recirculated part of the bottom product of the washing column is combined with the stream of washing agent partly loaded with methanol.

10. Use according to claim 8 or 9, wherein the washing apparatus furthermore comprises a condensation apparatus arranged in the upper region of the interior space, above the upper mass transfer zone and above the upper liquid distributor, preferably a reflux condenser.

## Revendications

1. Procédé pour la séparation et la récupération de méthanol contenu dans des gaz par lavage à l'eau, comprenant les étapes suivantes consistant à :
(a) fournir une colonne de lavage, comprenant une chemise qui (sur la base d'un agencement adéquat de la colonne de lavage) s'étend le long d'un axe longitudinal s'étendant parallèlement à la verticale et qui renferme un espace intérieur de la colonne de lavage, l'espace intérieur étant divisé en une région supérieure et une région inférieure, qui comprennent chacune au moins une zone de transfert de masse et qui sont séparées l'une de l'autre au moyen d'un plateau, en particulier un plateau à film, le plateau étant perméable à un écoulement gazeux dirigé de la zone de transfert de masse inférieure à la zone de transfert de masse supérieure, mais étant imperméable à un écoulement de liquide dirigé de la zone de transfert de masse supérieure à la zone de transfert de masse inférieure et étant conçu pour stocker temporairement un volume défini d'un agent de lavage s'écoulant hors de la zone de transfert de masse supérieure, la zone de transfert de masse supérieure et la zone de transfert de masse inférieure étant équipées de dispositifs de transfert de masse, de préférence des plateaux de séparation, des plateaux à tamis, des plateaux à coupelles, des plateaux à clapets, des garnitures structurées ou des lits fixes ou des combinaisons de ces dispositifs de transfert de masse,
(b) introduire de l'eau en tant qu'agent de lavage dans un premier échangeur de chaleur, refroidir l'agent de lavage dans le premier échangeur de chaleur, déverser un courant refroidi de l'agent de lavage depuis le premier échangeur de chaleur et introduire le courant refroidi de l'agent de lavage dans la région supérieure de la colonne de lavage, le point d'ajout étant agencé au-dessus de la zone de transfert de masse supérieure et étant désigné comme étant un distributeur de liquide,
(c) déverser un courant d'agent de lavage partiellement chargé en méthanol depuis le plateau de séparation et introduire le courant d'agent de lavage partiellement chargé en méthanol dans un second échangeur de chaleur, déverser le courant refroidi de l'agent de lavage partiellement chargé en méthanol depuis le second échangeur de chaleur et introduire le courant refroidi de l'agent de lavage partiellement chargé en méthanol dans la région inférieure de la colonne de lavage, le point d'ajout étant agencé au-dessous du plateau à film et au-dessus de la zone de transfert de masse inférieure et étant désigné comme étant un distributeur de liquide,
(d) déverser un courant d'agent de lavage chargé en méthanol depuis la région inférieure de la colonne de lavage sous la forme de son résidu, le point de soutirage du courant chargé de l'agent de lavage étant agencé au-dessous de la zone de transfert de masse inférieure,
(e) introduire un courant gazeux chargé en méthanol dans la région inférieure de la colonne de lavage, le point d'ajout étant agencé au-dessous de la zone de transfert de masse inférieure,
(f) déverser un courant gazeux à chargement de méthanol réduit depuis la région supérieure de la colonne de lavage sous la forme de son produit de tête, le point de soutirage du courant gazeux étant agencé au-dessus de la zone de transfert de masse supérieure.

2. Procédé selon la revendication 4, **caractérisé en ce qu'**au moins une partie du résidu de la colonne de lavage est remise en circulation vers le second échangeur de chaleur et combinée avec le courant d'agent de lavage partiellement chargé en méthanol.

3. Procédé selon la revendication 5, **caractérisé en ce que** la combinaison du résidu de la colonne de lavage avec le courant d'agent de lavage partiellement chargé en méthanol est effectuée avant d'introduire celui-ci dans le second échangeur de chaleur.

4. Procédé selon l'une quelconque des revendications précédentes, **caractérisé en ce qu'**une condensation de liquide hors du courant gazeux quittant la zone de transfert de masse supérieure est effectuée au moyen d'un appareil de condensation disposé dans la région supérieure de l'espace intérieur, au-dessus de la zone de transfert de masse supérieure et au-dessus du distributeur de liquide supérieur, de préférence un condenseur à reflux.

5. Procédé selon l'une quelconque des revendications précédentes, **caractérisé en ce qu'**il permet la séparation et la récupération de méthanol présent dans des effluents gazeux de cuves de stockage de méthanol ou dans des effluents gazeux de vases d'expansion de méthanol ou dans un gaz de purge de la synthèse du méthanol ou dans des combinaisons de ces effluents gazeux.

6. Procédé selon l'une quelconque des revendications précédentes, **caractérisé en ce que** de l'eau déminéralisée est utilisée comme agent de lavage.

7. Procédé selon l'une quelconque des revendications précédentes, **caractérisé en ce que** le résidu de la colonne de lavage est introduit dans un vase d'expansion de méthanol, une cuve de stockage de méthanol ou un appareil de distillation pour obtenir du méthanol pur.

8. Utilisation d'un appareil de lavage comprenant les éléments constitutifs et assemblages suivants :
(a) une colonne de lavage, comprenant une chemise qui (sur la base d'un agencement adéquat de la colonne de lavage) s'étend le long d'un axe longitudinal s'étendant parallèlement à la verticale et qui renferme un espace intérieur de la colonne de lavage, l'espace intérieur étant divisé en une région supérieure et une région inférieure, qui comprennent chacune au moins une zone de transfert de masse et qui sont séparées l'une de l'autre au moyen d'un plateau, en particulier un plateau à film, le plateau étant perméable à un écoulement gazeux dirigé de la zone de transfert de masse inférieure à la zone de transfert de masse supérieure, mais étant imperméable à un écoulement de liquide dirigé de la zone de transfert de masse supérieure à la zone de transfert de masse inférieure et étant conçu pour stocker temporairement un volume défini d'un agent de lavage s'écoulant hors de la zone de transfert de masse supérieure, la zone de transfert de masse supérieure et la zone de transfert de masse inférieure étant équipées de dispositifs de transfert de masse, de préférence des plateaux de séparation, des plateaux à tamis, des plateaux à coupelles, des plateaux à clapets, des garnitures structurées ou des lits fixes ou des combinaisons de ces dispositifs de transfert de masse,
(b) un premier échangeur de chaleur, un conduit pour introduire de l'eau en tant qu'agent de lavage dans le premier échangeur de chaleur, un conduit pour déverser un courant refroidi de l'agent de lavage depuis le premier échangeur de chaleur et pour introduire le courant refroidi de l'agent de lavage dans la région supérieure de la colonne de lavage, le point d'ajout étant agencé au-dessus de la zone de transfert de masse supérieure et étant désigné comme étant un distributeur de liquide,
(c) un second échangeur de chaleur, un conduit pour déverser un courant d'agent de lavage partiellement chargé en méthanol depuis le plateau de séparation et pour introduire le courant d'agent de lavage partiellement chargé en méthanol dans le second échangeur de chaleur, un conduit pour déverser le courant refroidi de l'agent de lavage partiellement chargé en méthanol depuis le second échangeur de chaleur et pour introduire le courant refroidi de l'agent de lavage partiellement chargé en méthanol dans la région inférieure de la colonne de lavage, le point d'ajout étant agencé au-dessous du plateau à film et au-dessus de la zone de transfert de masse inférieure et étant désigné comme étant un distributeur de liquide,
(d) un conduit pour déverser un courant d'agent de lavage chargé en méthanol depuis la région inférieure de la colonne de lavage sous la forme de son résidu, le point de soutirage du courant chargé de l'agent de lavage étant agencé au-dessous de la zone de transfert de masse inférieure,
(e) un conduit pour introduire un courant gazeux chargé en méthanol dans la région inférieure de la colonne de lavage, le point d'ajout étant agencé au-dessous de la zone de transfert de masse inférieure,
(f) un conduit pour déverser un courant gazeux à chargement de méthanol réduit depuis la région supérieure de la colonne de lavage sous la forme de son produit de tête, le point de soutirage du courant gazeux étant agencé au-dessus de la zone de transfert de masse supérieure,
dans un procédé selon les revendications 1 à 7 pour la séparation de méthanol contenu dans des gaz par lavage à l'eau.

9. Utilisation selon la revendication 8, l'appareil de lavage comprenant en outre un conduit pour la remise en circulation d'au moins une partie du résidu de la colonne de lavage vers le second échangeur de chaleur, la partie remise en circulation du résidu de la colonne de lavage étant combinée avec le courant de l'agent de lavage partiellement chargé en méthanol.

10. Utilisation selon la revendication 8 ou 9, dans laquelle l'appareil de lavage comprend en outre un appareil de condensation disposé dans la région supérieure de l'espace intérieur, au-dessus de la zone de transfert de masse supérieure et au-dessus du distributeur de liquide supérieur, de préférence un condenseur à reflux.
